# EUROPÄISCHE PATENTSCHRIFT

(11) **EP 1 881 763 B1**
(45) Veröffentlichungstag und Bekanntmachung des Hinweises auf die Patenterteilung: **06.04.2011**
(21) Anmeldenummer: 06742300.4
(22) Anmeldetag: 02.05.2006
(51) Int. Cl.: A01N 59/16, A01N 31/14, A01N 57/16, A01N 31/02, A01N 43/78, A01P 1/00, A01N 43/08

(54) **DEKONTAMINATIONSLÖSUNGEN UND DEREN VERWENDUNG ZUR DENATURIERUNG, MODIFIKATION, DEGRADATION, SOLUBILISIERUNG UND ENTFERNUNG VON PROTEINEN, NUKLEINSÄUREMOLEKÜLEN UND MIKROORGANISMEN**
DECONTAMINATION SOLUTIONS AND USE THEREOF FOR DENATURING, MODIFYING, DEGRADING, SOLUBILISING AND REMOVING PROTEINS, NUCLEIC ACID MOLECULES AND MICROORGANISMS
SOLUTIONS DE DECONTAMINATION ET LEUR UTILISATION POUR DENATURER, MODIFIER, DEGRADER, RENDRE SOLUBLES ET ELIMINER DES PROTEINES, MOLECULES D'ACIDE NUCLEIQUE ET MICRO-ORGANISMES

(30) Priorität: 30.04.2005 DE 102005020327
(43) Veröffentlichungstag der Anmeldung: 30.01.2008
(73) Patentinhaber: multiBIND biotec GmbH, 51105 Köln (DE)
(72) Erfinder: LISOWSKY, Thomas, 40789 Monheim (DE); ESSER, Karlheinz, 41199 Mönchengladbach (DE); LISOWSKY, Richard, 59174 Kamen (DE)
(74) Vertreter: Remus, Alvaro Johannes
(86) Internationale Anmeldenummer: PCT/DE2006/000758
(87) Internationale Veröffentlichungsnummer: WO 2006/116983

(56) Entgegenhaltungen:
- EP-A2- 0 998 937
- DE-A1- 19 936 428
- US-A- 4 797 274
- US-A1- 2004 234 621
- DATABASE WPI Week 200350 Derwent Publications Ltd., London, GB; AN 2003-532627 XP002420186 & WO 03/041499 A2 (ELIT HOLDING STOCK CO) 22. Mai 2003 (2003-05-22)

## Beschreibung

Die Erfindung betrifft eine Dekontaminationslösung zur Behandlung von Oberflächen, die mit unerwünschten Proteinen, Nukleinsäure-Molekülen oder Mikroorganismen kontaminiert sind. Die Erfindung betrifft ferner die Verwendung dieser Dekontaminationslösung und ein dafür geeignetes Puffersystem.

Durch die stürmischen Entwicklungen der Molekularbiologie werden Arbeitstechniken zum Nachweis und zur Amplifikation von DNA-Molekülen oder Proteinen immer wichtiger [Sambrook, J. et al., eds (1989) Molecular Cloning: A Laboratory Manual, 2nd Led., Cold Spring Harbor Laboratory Press, Cold Spring Harbor, NY.]. Aktuelle Beispiele betreffen z.B. die medizinische Diagnostik, forensische Nachweisverfahren und biomedizinische Forschungen.

Mit der Entdeckung der Polymerase-Ketten-Reaktion (PCR) sind sogar Einzelmolekülnachweise möglich. Ein neues Problem dieser extremen Verfeinerung der Nachweismethoden ist die Kontamination von Oberflächen in Laboren, an Geräten oder Arbeitsmaterialien mit unerwünschten Nukleinsäuremolekülen, Proteinen oder Mikroorganismen.

Mikrobielle Kontaminationen verursachen weiterhin große Probleme und kommerzielle Verluste zum Beispiel im Bereich der Lebensmitteltechnologie, in der Produktion, in Krankenhäuser, Hygieneinstituten und auch im Haushalt.

### Stand der Technik

Schon länger gibt es daher verschiedenste Dekontaminationslösungen mit aggressiven chemischen Substanzen wie z.B. Formaldehyd, Alkoholen, Phenolen, Natriumazid, Natriummhypochlorid gegen Mikroorganismen oder stark oxidierenden Agenzien, wie z.B. Hypochlorid, Bleichmitteln oder mineralischen Säuren, die Proteine denaturieren und Nukleinsäuren modifizieren und unamplifizierbar machen für Amplifizierungsmethoden wie z.B. Polymerase-Kettenreaktion (PCR), Nicktranslation mit Klenow-Polymerase, Strangverdrängungs-Amplifikation, Ligase-Kettenreaktion, transkriptionsvermittelte Amplifikation, Rolling-Circle-Amplifikation und viele andere mehr.

Die zur Dekontamination verwendeten stark aggressiven Chemikalien und oxidierenden Agenzien verursachen dabei eine permanente Modifizierung und Denaturierung der Proteine und Destabilisierung des Doppelstranges und Modifikationen die eine Amplifikation blockieren. Im Allgemeinen ergeben sich dabei Modifikationen an besonders reaktiven Gruppen der Moleküle und oxidative Schädigungen.

Deshalb werden allgemein solche aggressiven chemischen Lösungen zur Waschung und Spülung von Geräten, Instrumenten und Arbeitsoberflächen verwendet.

Nachteile dieser Lösungen und Methoden sind dabei die jeweils nur selektiven Wirksamkeiten gegen Proteine, DNA oder Mikroorganismen und die unvollständige Entfernung aller Nukleinsäuren, der Erhalt der modifizierten Moleküle, der nur teilweise Abbau und die Aggressivität der verwendeten Chemikalien gegenüber Geräten, Instrumenten, Oberflächen und auch der Haut und Schleimhäute der Anwender.

Eine graduelle Verbesserung der Effizienz dieser Methode brachte die Kombination der Lösungen mit oberflächenaktiven Substanzen. Aber auch hierbei bleiben die aggressiven chemischen Substanzen und die unvollständige Zerstörung und Entfernung der Nukleinsäuren, Proteine und Mikroorganismen problematisch.

Das kommerzielle Interesse an solchen Lösungen unterstreicht der bereits breite kommerzielle Vertrieb von entsprechenden DNA- und Protein-Dekontaminations-Lösungen und antimikrobiellen Mitteln unter den verschiedensten Markennamen.

Aus der DE-A-19 936 428 ist beispielsweise ein Bakterizid bekannt, dass Eisen(III)-Ionen (Fe³⁺), L-Ascorbinsäure und ein oder mehrere Mitglieder aus der Gruppe bestehend aus Sorbinsäure, Benzoesäure und para-Hydroxybenzoesäureester enthält. Die bakterizide Wirkung dieser Zusammensetzung kann beispielsweise durch Zugabe einer kleinen Menge eines grenzflächenaktiven Mittels verstärkt werden.

Die Nachteile dieser bekannten Dekontaminationslösungen und Methoden sind ihre nur begrenzte Wirkungen gegen verschiedenartige biologische Moleküle wie entweder Proteine oder Nukleinsäuren oder nur antimikrobielle Wirkung und ihre zum Teil sehr aggressiven chemischen Wirkungen mit gesundheitsschädlichen Eigenschaften. Besonders für die Dekontamination von Mikroorganismen gibt es bis heute nur Mittel die eine Abtötung bewirken, aber die Erbinformation, extrachromosomale Erbträger und Proteine nicht inaktivieren oder zerstören.

Es ist bekannt, dass in physiologischen Mengen von mikromolaren Konzentrationen Antioxidanzien in Kombination mit zweiwertigen Metallionen vereinzelt zu Schäden und partiellen Strangbrüchen an Nukleinsäuremolekülen führen können (Padayatty S.J., Katz A., Wang Y., Eck P., Kwon O., Lee J.H., Chen S., Corpe C, Dutta A, Dutta SK and Levine M. (2003) Vitamin C as an antioxidant: evaluation of its role in disease prevention. J. Am. Coll. Nutr. 1, 18-35; Blokhina O., Virolainen E., Fagerstedt K.V. (2003) Antioxidants, Oxidative Damage and Oxygen Depriviation Stress: a Review. Annals Botany 91:179-194; Veal J. M., Merchant K. & Rill R. L. (1991) The influence of reducing agent and 1,10-phenanthroline concentration on DNA cleavage by phenanthroline + copper. Nucl Acids Res Vol. 19, No. 12, 3383-3388). Dies sind jedoch nur vereinzelte, isolierte Ergebnisse, die nur auf den jeweiligen Einzelfall anwendbar sind.

Die neuen Erkenntnisse der modernen Molekularbiologie und Gentechnologie zeigen, dass schon die Erbinformation alleine, einzelne Gene oder sogar Teile davon, sowie bestimmte Proteine ausreichen, um Erkrankungen auszulösen oder unerwünschte genetische Veränderungen zu verursachen.

Daher besteht in der Praxis ein Bedarf an verbesserten Verfahren, Agenzien, Methoden und Lösungen zur effizienten und vor allem auch schonenden vollständigen Dekontamination von Oberflächen und Geräten von Proteinen, Nukleinsäuren und Mikroorganismen.

### Zusammenfassung der Erfindung

Aufgabe der vorliegenden Erfindung ist es daher, die Nachteile des Standes der Technik zu überwinden und neue Verfahren und Lösungen zu entwickeln, die nicht mit aggressiven Chemikalien oder stark oxidierenden Agenzien arbeiten und außerdem die behandelten Substrate vollständig dekontaminieren.

Diese Aufgabe wird erfindungsgemäß gelöst durch eine Dekontaminationslösung, die zumindest eine synergistische Mischung aus
a) mindestens einem Vitamin in Mengen von 10 mM bis 100 mM,
b) mindestens einem Metallion in Mengen von 1 mM bis 100 mM und
c) mindestens einer oberflächenaktiven Verbindung in Mengen von 0.1 % bis 10 Gew.-% der Gesamtlösung enthält.

Bei der Verwendung natürlicher Antioxidanzien in Kombination mit Metallionen und oberflächenaktiven Stoffen hat sich überraschenderweise herausgestellt, dass verschiedenste Vitamine in Kombination mit Metallionen und Detergenzien zu extrem schnellen, massiven Strangbrüchen und Modifikationen in Nukleinsäuremolekülen und Proteinen führen. Dieser überraschende Effekt führt zu einer effizienten Abtötung von Mikroorganismen durch die Inaktivierung und Zerstörung ihrer Erbinformation und Proteine. Besonders überraschend und neu ist der Befund, dass mit dem erfindungsgemäßen Dreikomponenten-System die Inaktivierung und Degradation im gesamten pH-Bereich von 2 bis 8,5 mit im Wesentlichen gleicher Effizienz abläuft. Dadurch, dass in einem vergleichsweise milden pH-Bereich gearbeitet werden kann, schont die erfindungsgemäße Lösung in vorteilhafter Weise die zu behandelnden Oberflächen und ist darüber hinaus hautverträglich für den Anwender. Durch Aufsprühen, Verreiben oder Einweichen in der Lösung der drei Komponenten werden Proteine und Nukleinsäuren denaturiert, solubilisiert, inaktiviert, degradiert und entfernt und damit dann auch Mikroorganismen sehr effizient abgetötet.

In vorteilhafter Ausgestaltung der Erfindung ist vorgesehen, dass die Mischung einen pH-Wert im Bereich von 3 bis 7, vorzugsweise von 4 bis 6, aufweist. In diesen pH-Bereichen ist die erfindungsgemäße Lösung über längere Zeiträume stabil und ermöglichen einen besonders effektiven Abbau von Nukleinsäuren. Darüber hinaus ist im Bereich von pH 4 bis 6 die Hautverträglichkeit der erfindungsgemäßen Lösung optimal.

Besonders bevorzugt ist eine vorteilhafte Ausführungsform, bei der die Mischung zusätzlich ein Puffersystem mit Carbonaten und Derivaten der Bernsteinsäure, vorzugsweise jeweils in einer Konzentration von 1 mM bis 500 mM, enthält. Bei der Verwendung dieses erfindungsgemäßen Puffersystems in der erfindungsgemäßen Dekontaminationslösung kann der pH-Wert der Lösung, der sich aufgrund der gelösten Komponenten, insbesondere der sauren Vitamine, im stark sauren Bereich befindet, leicht bis beispielsweise in den neutralen oder schwach basischen Bereich angehoben werden, ohne dass die gelösten Metallionen ausfallen.

In einer bevorzugten Ausführungsform der erfindungsgemäßen Dekontaminationslösung sind die erfindungsgemäß enthaltenen Vitamine bzw. deren Salze oder sauren Derivate ein oder mehrere Verbindungen und/oder deren Salze ausgesucht aus der Gruppe der wasserlöslichen Vitamine mit den Eigenschaften von Antioxidanzien, wie vorzugsweise Vitamin C, Riboflavin und Niacin. Sie werden in Mengen von 10 mM bis 100 mM bezogen auf die Gesamtlösung eingesetzt.

In einer weiteren bevorzugten Ausführungsform der erfindungsgemäßen Dekontaminationslösung sind die erfindungsgemäß enthaltenen Metallionen zwei und/oder 3-wertige Ionen der Metalle der 4. Periode und/oder Nebengruppen I, II und VIII des periodischen Systems der Elemente. Sie werden in Form ihrer Salze mit organischen und/oder anorganische Säuren oder Basen eingesetzt. Erfindungsgemäß bevorzugt sind ein oder mehrere Verbindungen ausgesucht aus der Nebengruppe VIII, insbesondere Eisen, Kobalt, Nickel, Kupfer oder Zink. Sie werden in Mengen von 1 mM bis 100 mM, bezogen auf die Gesamtlösung, insbesondere in Mengen von 5 mM bis 10 mM, eingesetzt.

Die erfindungsgemäß enthaltenen oberflächenaktiven Substanzen können beispielsweise anionische, nichtionische, amphotere oder kationische inerte Tenside oder geeignete Mischungen miteinander oder untereinander sein. Insbesondere können Alkylethersulfate, Alkyl- und/oder Arylsulfonate, Alkylsulfate, Amphotenside, Betaine, Alkylamidoalkylamine, Alkylsubstituierte Aminosäuren, alkylsubstituierte Iminosäuren, Acylierte Aminosäuren, Amphotensidkombinationen eingesetzt werden. Grundsätzlich sind alle inerten Tenside geeignet. Inert bedeutet, dass sie weder die synergistische Lösung noch das Versuchsergebnis beeinflussen. Erfindungsgemäß bevorzugt sind anionische und nichtionische Tenside. Sie werden in Mengen von 0.1 %. bis 10 Gew.-%, bezogen auf die Gesamtlösung, insbesondere in Mengen von 0.2 % bis 0.5 Gew.-%, eingesetzt.

Die erfindungsgemäßen Dekontaminationslösungen können zusätzlich weitere übliche inerte Hilfs- und Zusatzstoffe enthalten wie beispielsweise geeignete Puffersubstanzen zur Einstellung eines definierten pH-Wertes, wie beispielsweise Tris (Tris(hydroxymethyl)-aminomethan), MES (2(Morpholino)ethansulfonsäure), HEPES (2-[4-(2-Hydroxyethyl)-1-piperazinyl]-ethansulfonsäure und/oder MOPS (3-(N-Morpholino) propansulfonsäure. Diese Puffersubstanzen werden in Mengen von 1 mM bis 500 mM bezogen auf die Gesamtlösung eingesetzt.

Die effiziente Wirkung des neuen Dreikomponenten-Systems ist umso überraschender, da nachgewiesenermaßen die jeweiligen Einzelkomponenten keine besondere degradierende Wirkung zeigen und auch Mischungsverhältnisse außerhalb des erfindungsgemäßen Bereiches nicht oder nur unzureichend wirken. Erst die Kombination von Vitaminen mit Metallionen und Detergenzien, vorzugsweise in einem geeigneten Mischungsverhältnis, führt zu einem synergistischen Effekt und zu einem sehr schnellen und massiven Abbau der Biomoleküle. Insbesondere bei Einhaltung der korrekten bevorzugten Konzentrationen ist eine effektive Wirksamkeit der erfindungsgemäßen Dekontaminationslösung gewährleistet. Grundsätzlich können so alle Oberflächen sehr schonend zur Entfernung von Proteinen, Nukleinsäure-Kontaminationen und Mikroorganismen behandelt werden.

Die Dekontaminierung erfolgt in der Regel durch Aufsprühen oder Verreiben der erfindungsgemäßen Lösung auf kontaminierte Oberflächen oder durch Einweichen. Als Einwirkzeit sind 0,5 bis 2 Minuten bei Raumtemperatur oder schwach erhöhter Temperatur im allgemeinen ausreichend zur vollständigen Denaturierung, Modifikation, Degradation, Solubilisierung und Entfernung von Proteinen, Nukleinsäuremolekülen und Mikroorganismen von Oberflächen. Die angewandten Verfahren sind jedoch variierbar und können den jeweiligen Erfordernissen angepasst werden.

Ein weiterer Gegenstand der Erfindung ist die Verwendung der erfindungsgemäßen Dekontaminationslösungen zur Denaturierung, Modifikation, Degradation, Solubilisierung und Entfernung von Proteinen und Nukleinsäuremolekülen von Oberflächen.

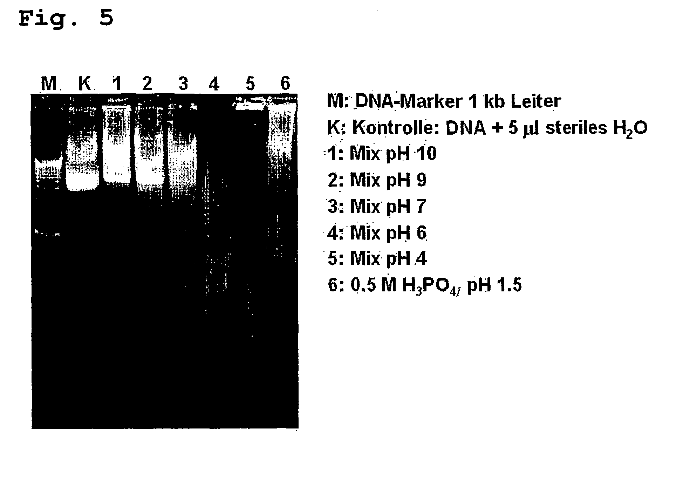

Das neue, vorteilhafte Puffersystem mit Carbonaten und Derivaten der Bernsteinsäure ist für die erfindungsgemäße Dekontaminationslösung besonders geeignet. Die verschiedenen Mischungen mit pH-Werten zwischen pH 2 und 8,5 ergeben jeweils klare leicht gelbliche bis bräunliche Lösungen, die über längere Zeiträume stabil sind und insbesondere im Bereich pH 4 bis pH 6 einen sehr effizienten Abbau der DNA-Moleküle bewirken, wie der Vergleich mit der mineralischen 0.5 M Phosphorsäure von pH 1.5 in Figur 5 zeigt.

In vorteilhafter Ausgestaltung des erfindungsgemäßen Puffersystems ist vorgesehen, dass die Carbonate und Derivate der Bernsteinsäure jeweils in einer Konzentration von 1 mM bis 500 mM enthalten sind.

Die Lösung mit dem Puffersystem kann in vorteilhafter Weise auf einen pH-Wert im Bereich von 2 bis 8,5, insbesondere von 3 bis 7, vorzugsweise von 4 bis 6, eingestellt sein. Hierdurch wird gewährleistet, dass die weiteren in der Lösung gelösten Bestandteile nicht beeinträchtigt werden und darüber hinaus eine hautverträgliche Lösung hergestellt werden kann.

Die Erfindung betrifft daher auch ein Verfahren zum Einstellen des pH-Wertes einer Lösung, die zumindest eine Mischung aus
a) mindestens einem Vitamin in Mengen von 10 mM bis 100 mM,
b) mindestens einem Metallion in Mengen von 1 mM bis 100 mM und
c) mindestens einer oberflächenaktiven Verbindung in Mengen von 0.1 % bis 10 Gew.-% der Gesamtlösung enthält, wobei die Mischung mittels des erfindungsgemäßen Puffersystems auf einen pH-Wert im Bereich von 2 bis 8,5, insbesondere von 3 bis 7, vorzugsweise von 4 bis 6, eingestellt werden kann.

### Kurze Beschreibung der Abbildungen

Die Erfindung wird durch die folgenden nicht-einschränkenden Figuren, Beispiele und Tabellen veranschaulicht:
Dabei zeigen **Fig. 1 bis 5** die effiziente Degradation von DNA-Molekülen durch das neue Dreikomponenten-System im Vergleich mit bekannten anderen Lösungen.
**Fig. 6** zeigt die Blockierung der PCR-Amplifizierbarkeit von DNA-Molekülen nach der Behandlung mit dem neuen Dreikomponenten-System.
**Fig. 7** zeigt den Standard-Test mit RNaseA zur Inaktivierung von Enzymen durch das neue Dreikomponenten-System.
**Fig. 8**. zeigt den effizienten Abbau genomischer DNA und extrachromosomaler Erbträger in Mikroorganismen durch das neue Dreikomponenten-System.
**Tabelle 1** zeigt den Test zur antimikrobiellen Wirkung des neuen Dreikomponenten-Systems.
**Tabelle 2** zeigt den bevorzugten Grundaufbau und die bevorzugten Zusammensetzungen für das Dreikomonenten-System aus Detergenz, Vitaminen und Metallionen.

### Beschreibung vorteilhafter und bevorzugter Ausführungsformen der Erfindung

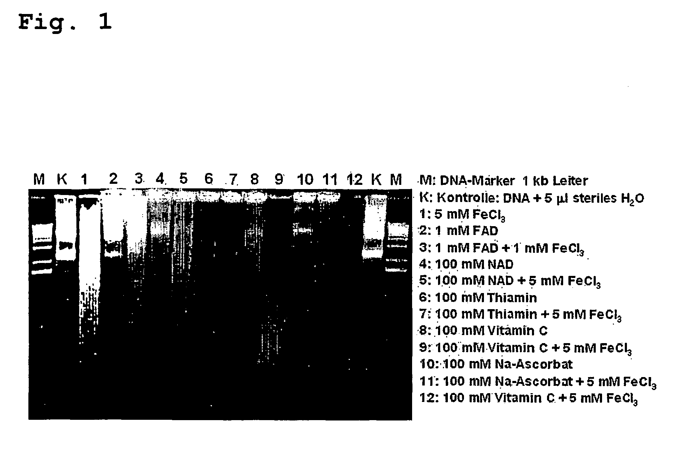

**Figuren 1 bis 5** zeigen die effiziente Degradation von DNA-Molekülen durch das neue Dreikomponenten-System im Vergleich mit bekannten anderen Lösungen. Identische Aliquots von DNA-Plasmiden (YEp351) wurden für 2 Minuten mit den aufgeführten Lösungen behandelt. Anschließend wurden die DNA-Proben denaturiert und die einzelsträngigen DNA-Moleküle auf einem Agarosegel (1%) gelelektrophoretisch aufgetrennt. Nach der Färbung mit Ethidiumbromid erhält man die aufgeführten Bilder. Die Kontrolle zeigt die intakte Plasmid-DNA nach Behandlung mit sterilem Wasser. Bei der Einfügung von Strangbrüchen verkleinert sich das Molekulargewicht der betroffenen DNA-Moleküle. Dies kann im Gel durch den Vergleich mit der Kontrolle und mit dem Molekulargewichtsmarker bestimmt werden. Es wurden jeweils 5 µg DNA in 5 µl sterilem Tris-Puffer(1 mM; pH 8.0) mit 5 µl der angegebenen Lösungen für 2 Minuten bei Raumtemperatur behandelt. Anschließend wurden die Proben mit 5 µl 100 mM Tris (pH 12) gemischt, mit Bromphenolblau-Marker versehen und für 5 Minuten bei 95°C denaturiert. Die denaturierten Proben wurden sofort auf 4°C abgekühlt und jeweils Aliquots mit 1 µg DNA pro Gelspur aufgetragen.

Die DNA wurde nach der Gelelektrophorese im 1%-igen Agarosegel mit Ethidiumbromid angefärbt und fotografiert.

**Fig. 1** zeigt den Vergleich des Nukleinsäure-Abbaus durch Vitamine, Metallionen und ein Dreikomponenten-System aus Vitaminen, Metallionen und Detergentien. (M: DNA-Marker 1 kb Leiter; K: Kontrolle: DNA + 5 ml steriles H2O; 1:5 mM FeCl32: 1 mM FAD; 3: 1 mM FAD + 1 mM FeCl3; 4: 100 mM NAD; 5: 100 mM NAD + 5 mM FeCl3; 6: 100 mM Thiamin; 7: 100 mM Thiamin + 5 mM FeCl3; 8: 100 mM Vitamin C; 9: 100 mM Vitamin C + 5 mM FeCl3; 10: 100 mM Na-Ascorbat; 11: 100 mM Na-Ascorbat + 5 mM FeCl3; 12: 100 mM Ascorbinsäure + 5 mM FeCl₃). Alle Proben mit je 0,2 % Triton-100 und 0,2 % Tween 20.

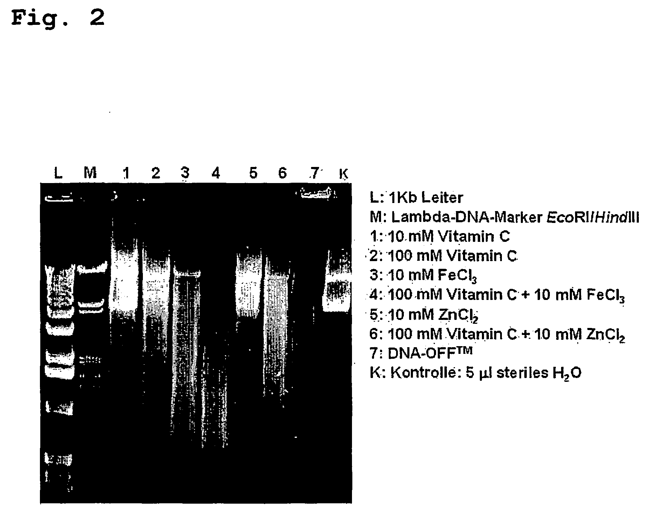

**Fig. 2** zeigt den Test des Nukleinsäure-Abbaus durch Kombinationen aus Vitamin C, Metallionen und Detergentien. L: 1kb Leiter; M: Lambda-DNA-Marker *EcoRI*/*HindIII;* 1: 10 mM Vitamin C; 2: 100 mM Vitamin C; 3: 10 mM FeCl₃; 4: 100 mM Vitamin C + 10 mM FeCl₃; 5: 10 mM ZnCl₂; 6: 100 mM Vitamin C + 10 mM ZnCl₂; 7: DNA-OFFTM; K: Kontrolle: 5 µl steriles H₂O). Alle Proben mit je 0,2 % TritonX-100 und 0,2 % Tween 20.

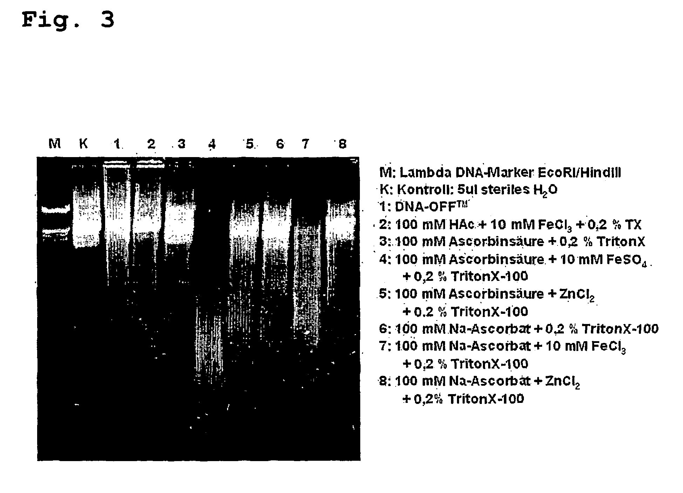

**Fig. 3** zeigt Vergleich des Nukleinsäure-Abbaus durch Mischungen mit Ascorbinsäure oder Na-Ascorbat. (M: Lambda DNA--Marker *Eco*RI/*Hind*III; K: Kontroll: 5 µl steriles H2O; 1: DNA-OFF^{™}; 2: 100 mM HAc + 10 mM FeCl3 + 0,2 % TritonX-100; 3: 100 mM Ascorbinsäure + 0,2 % Triton-100; 4: 100 mM Ascorbinsäure + 10 mM FeSO4 + 0,2 % TritonX-100; 5: 100 mM Ascorbinsäure + ZnCl2 + 0.2 % TritonX-100; 6: 100 mM Na-Ascorbat + 0,2 % TritonX-100; 7: 100 mM Na-Ascorbat + 10 mM FeCl3 + 0,2 % TritonX-100; 8: 100 mM Na-Ascorbat + ZnCl2 + 0,2% TritonX-100).

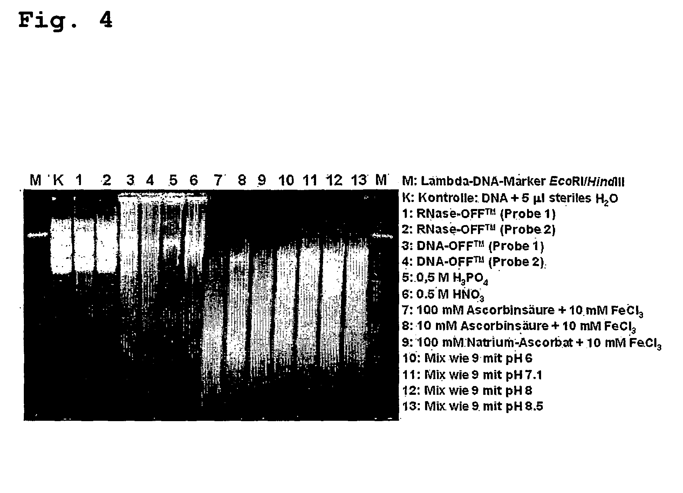

**Fig. 4** zeigt Vergleich des Nukleinsäure-Abbaus durch Mischungen mit mineralischen Säuren, Ascorbinsäure oder Na-Ascorbat (pH 6 bis 8,5). (M: Lambda-DNA-Marker *Eco*RI/*Hind*III K: Kontrolle: DNA + 5 µl steriles H2O; 1: RNase-OFFTM (Probe 1); 2: RNase-OFFTM (Probe 2); 3: DNA-OFFTM (Probe 1); 4: DNA-OFFTM (Probe 2); 5: 0,5 M H3PO4; 6: 0.5 M HNO3; 7: 100 mM Ascorbinsäure + 10 mM FeCl3; 8: 10 mM Ascorbinsäure + 10 mM FeC13; 9: 100 mM Natrium-Ascorbat + 10 mM FeCl3; 10: Mix wie 9 nur mit pH 6; 11: Mix wie 9 nur mit pH 7.1; 12: Mix wie 9 nur mit pH 8; 13: Mix wie 9 nur mit pH 8.5). Die Proben 5 bis 13 enthalten je 0,2 % TritonX-100 und 0,2% Tween 20.

**Fig. 5** zeigt ein Beispiel für ein neues Puffersystem mit Natriumcarbonat und Hydroxybernsteinsäure im Vergleich mit mineralischer Säure. Die Grundmischung enthielt 50 mM Ascorbinsäure, 5 mM FeCl₃ und je 0,2 % TritonX-100 und 0,2% Tween 20. (M: DNA-Marker 1 Kb Leiter), K: Kontrolle: DNA + 5 µl steriles H₂O; 1: Mischung pH 10; 2: Mischung pH 9; 3: Mischung pH 7; 4: Mischung pH 6; 5: Mischung pH 4; 6: 0.5 M H₃PO₄ (pH 1.5) mit 0,2 % TritonX-100 und 0,2% Tween 20.

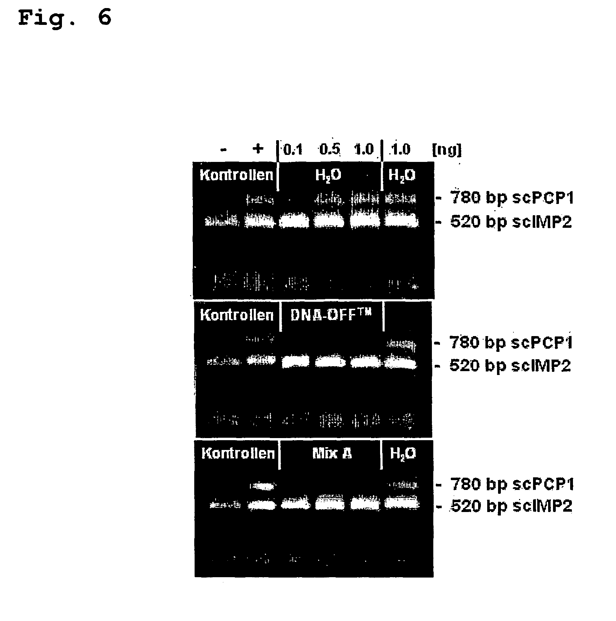

**Fig. 6** zeigt die Blockierung der PCR-Amplifizierbarkeit von DNA-Molekülen nach der Behandlung mit dem neuen Dreikomponenten-System (Mix A: 100 mM Vitamin C + 10 mM FeCl₃+ 0.2 % TritonX-100 und 0,2 % Tween 20). Verschiedene Mengen (0.1 bis 1 ng) einer DNA-Probe wurden in PCR-Gefäßen getrocknet. Diese PCR-Gefäße mit der eingetrockneten DNA wurden für 20 Sekunden mit den aufgeführten Lösungen behandelt. Anschließend wurden die Gefäße 2x mit je 100 µl sterilem destilliertem Wasser gewaschen. Dann wurden die Gefäße mit einem 50 µl PCR-Reaktionsmix gefüllt und die PCR-Reaktion durchgeführt. Der PCR-Reaktionsmix enthält Primerpaare für die Amplifikation der Kontroll-DNA (scIMP2-Gen der Hefe) und der Test-DNA (scPCP1-Gen der Hefe). Die Kontroll-DNA (1ng) zeigt an, ob die PCR-Reaktion erfolgreich verlaufen ist. Eine Bande der Test-DNA zeigt an, dass noch intakte DNA-Moleküle für dieses Gens vorhanden sind. Bei einer vollständigen Entfernung oder Blockierung der Test-DNA darf es keine amplifizierte DNA-Bande im Gel geben. Die DNA wurde nach der Gelelektrophorese im 1%-ige Agarosegel mit Ethidiumbromid angefärbt und fotografiert. Als positive Kontrolle dient das bekannte DNA-OFF^{™}. Als negative Kontrolle wird steriles Wasser (H₂O) verwendet.

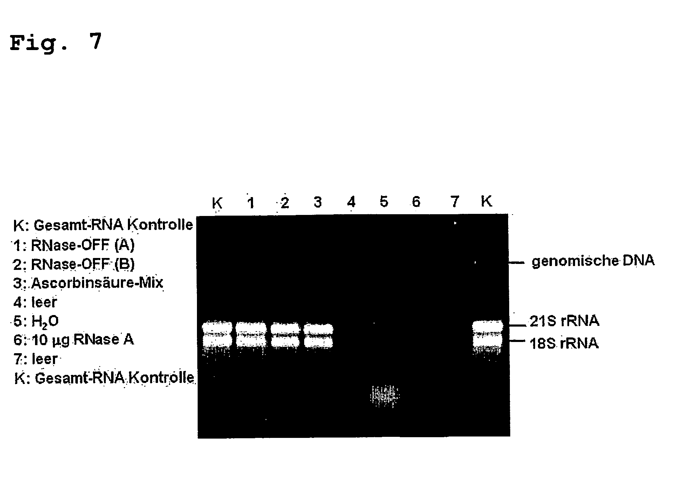

**Fig. 7** zeigt den Standard-Test mit RNaseA zur Inaktivierung von Enzymen durch das neue Dreikomponenten-System. Identische Aliquots von 10 µg RNaseA wurden in Eppendorfgefäßen eingetrocknet. Dann wurden diese Gefäße mit je 1 ml aufgeführten Lösungen versehen, 20 Sekunden gevortext und dann 5 Minuten bei RT inkubiert. Anschließend wurden die Gefäße 2-mal mit je 1 ml sterilem Wasser gewaschen. Dann wurden je 5 µg Gesamt-RNA von *E*. *coli* in die Gefäße gegeben und für 30 Minuten bei 37°C inkubiert. Anschließend wurden die Gesamt-RNA-Proben mit Formamid/Bromphenolblau-Puffer versehen und bei 95°C für 5 Minuten denaturiert. Anschließend wurden die kompletten 5 µg Gesamt-RNA in einem Agarosegel (1,2%) gelelektrophoretisch aufgetrennt. Nach der Färbung mit EtBr erhält man das aufgeführte Bild. Die unbehandelte Kontrollen zeigen die intakte Gesamt-RNA. Bei der Anwesenheit von aktiver RNaseA werden diese RNA-Moleküle abgebaut. Bei erfolgreicher vollständiger Inaktivierung der RNaseA bleiben die Gesamt-RNA-Moleküle gleichfalls unverändert.

(K: Gesamt-RNA Kontrolle; 1: RNase-OFF (A); 2: RNase-OFF (B); 3: Ascorbinsäure-Mix mit 100 mM Vitamin C + 10 mM FeCl₃+ 0.5 % SDS; 4: leer; 5: H2O; 6: 10 µg RNase A; 7: leer; K: Gesamt-RNA Kontrolle)

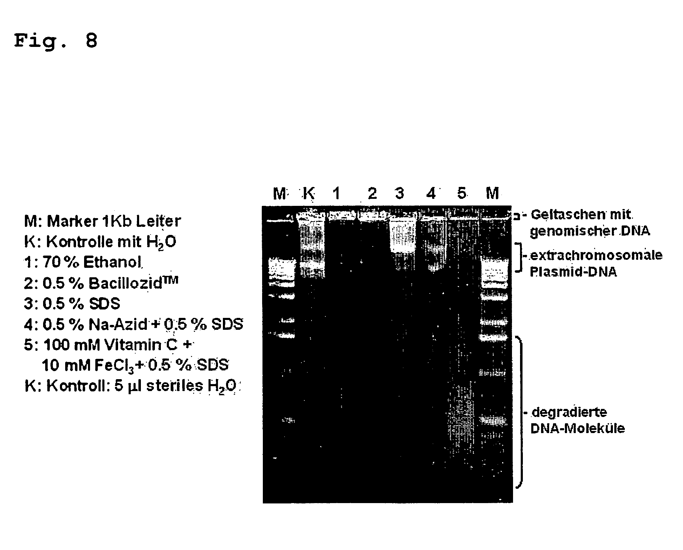

**Fig. 8****.** zeigt den effizienten Abbau genomischer DNA und extrachromosomaler Erbträger in Mikroorganismen durch das neue Dreikomponenten-System. Ein rekombinanter *Escherichia coli* Stamm mit einem extrachromosomalen Plasmid (YEp351) wurde über Nacht in LB amp Medium angezogen. Je 5 µl dieser *E. coli* Suspension wurden mit 5 µl Lysozymlösung (1mg/ml) für 5 Minuten behandelt und dann mit 5 µl der aufgeführten Lösungen (1 - 5) für weitere 5 Minuten inkubiert. Nach Zugabe von Bromphenolblau wurden die Proben in die Geltaschen gegeben und durch Elektrophorese die DNA-Moleküle aufgetrennt. Nur in der Probe 5 mit dem Dreikomponenten-System ist eine massive Degradation der DNA-Moleküle nachweisbar. Bei der Kontrolle mit sterilem Wasser (K) und bei der Probe 3 und 4 wird eine Lyse der Zellen beobachtet, so dass die extrachromosomal Plasmid-DNA freigesetzt wird und in das Gel einwandern kann. In den Proben 1 und 2 beobachtet man eine Ausfällung des Zelllysates und der DNA, so dass alle DNA-Moleküle in der Geltasche liegen bleiben.

(M: Marker 1Kb Leiter; K: Kontrolle mit H₂O; 1: 70 % Ethanol; 2: 0.5 % Bacillozid^{™}; 3: 0.5 % SDS; 4: 0.5 % Na-Azid + 0.5 % SDS; 5: 100 mM Vitamin C + 10 mM FeCl₃+ 0.5 % SDS; K: Kontroll: 5 µl steriles H₂O)

**Tabelle 1** zeigt den Test zur antimikrobiellen Wirkung des neuen Dreikomponenten-Systems.

Frische Kulturen der angegeben Mikroorganismen wurden auf eine Zellzahl von 10⁶ in 50 µl eingestellt und dann mit je 50 µl Wasser, 70 % Ethanol oder dem Dreikomponenten-System (100 mM Ascorbinsäure, 10 mM FeCl₃ und 0.5 % SDS) im Verhältnis 1:1 gemischt. Nach einer Inkubationszeit von 2 Minuten wurden die 100 µl Ansätze mit den Bakterien auf entsprechende Wachstumsplatten ausplattiert. Nach einer Inkubation von 1 - 3 Tagen bei 28°C (*Saccharomyces cerevisiae* und *Candida parapsilosis*) oder 37°C (*Escherichia coli* und *Bacillus subtilis*) wurde die Zahl der gewachsenen Kolonien bestimmt. In den Ansätzen mit sterilem Wasser überlebten alle Mikroorganismen. Die Ansätze mit 70 % Ethanol oder dem Dreikomponenten-System zeigten kein Koloniewachstum, was anzeigt, dass in diesen Fällen alle Mikroorganismen abgetötet wurden.

**Tabelle 2** zeigt die bevorzugte Zusammensetzungen der Lösung mit Dreikomponenten aus oberflächen-aktiver Substanz, Vitaminen und Metallionen für die Entfernung von DNA-Molekülen von Oberflächen und Geräten.

### Liste mit Erläuterung der Abkürzungen in den Figuren

amp: Ampicillin
Bacillozid^{™}: kommerzielle, antibakterielle Lösung
DNase-OFF^{™}:kommerzielle Lösung zur Inaktivierung von DNA
EtBr: Ethidiumbromid
FAD: Flavinadenindinukleotid
K: Kontrolle
M: Molekulargewichtsmarker
PCR: Polymerase Chain Reaktion / Polymerasekettenreaktion
RNase-OFF™:kommerzielle Lösung zur Inaktivierung von RNasen
RNaseA: Ribonuklease A (aus Rinderpankreas)
RT: Raumtepmeratur
sc: *Saccharomyces cerevisiae*
scIMP2: *Saccharomyces cerevisiae* Gen
   für Inner Membrane Protease 2
scPCP1: *Saccharomyces cerevisiae* Gen
   für Processing of Cytochrome c Peroxidase
SDS: Sodiumdodecylsulfate
TX: TritonX-100 (nichtionisches Detergenz)
YEp351: Yeast Episomal plasmid

**Tabelle 1**

| | | | |
|---|---|---|---|
| Test zur antimikrobiellen Wirkung des neuen Dreikomponenten-Systems. | | | |

| | H₂O | 70% Ethanol | Dreikomponenten-System |
|---|---|---|---|
| Mikroorganismen | | | |
| *Escherichia coli* | 10⁶ | 0 | 0 |
| *Bacillus subtilis* | 10⁶ | 0 | 0 |
| *Saccharomyces cerevisiae* | 10⁶ | 0 | 0 |
| *Candida parapsilosis* | 10⁶ | 0 | 0 |

**Tabelle 2**

| |
|---|
| Bevorzugte Zusammensetzungen der Lösung mit Dreikomponenten aus oberflächen-aktiver Substanz, Vitaminen und Metallionen für die Entfernung von DNA-Molekülen von Oberflächen und Geräten. |
| Lössungsansätze |
| pH Bereich: 2.0 - 8.5 |
| Vitamine: 1 mM - 100 mM |
| Metallionen: 1 mM bis 50 mM |
| Detergenzien: 0.1 % bis 5 % |

## Patentansprüche

1. Dekontaminationslösung, zumindest enthaltend eine synergistische Mischung aus
a) mindestens einem Vitamin in Mengen von 10 mM bis 100 mM,
b) mindestens einem Metallion in Mengen von 1 mM bis 100 mM und
c) mindestens einer oberflächenaktiven Verbindung in Mengen von 0.1 % bis 10 Gew.-% der Gesamtlösung.

2. Dekontaminationslösung gemäß Anspruch 1, **dadurch gekennzeichnet, dass** die Mischung einen pH-Wert im Bereich von 2 bis 8.5 aufweist.

3. Dekontaminationslösung gemäß Anspruch 1 oder 2, **dadurch gekennzeichnet, dass** die Mischung zusätzlich ein Puffersystem mit Carbonaten und Derivaten der Bernsteinsäure enthält.

4. Dekontaminationslösung gemäß mindestens einem der Ansprüche 1 bis 3, **dadurch gekennzeichnet, dass** als Vitamine, deren Salze oder sauren Derivate, mindestens eine Verbindung, ausgesucht aus der Gruppe der wasserlöslichen Vitamine mit der Eigenschaft von Antioxidanzien, enthalten sind.

5. Dekontaminationslösung gemäß mindestens einem der Ansprüche 1 bis 4, **dadurch gekennzeichnet, dass** die Metallionen aus den Metallen der 4. Periode und der Nebengruppen I, II, oder VIII des periodischen Systems der Elemente stammen.

6. Dekontaminationslösung gemäß Anspruch 5, **dadurch gekennzeichnet, dass** die Metalle in Form ihrer Salze mit Säuren oder Basen vorliegen.

7. Dekontaminationslösung gemäß mindestens einem der Ansprüche 1 bis 6, **dadurch gekennzeichnet, dass** als oberflächenaktive Substanz mindestens eine Verbindung ausgesucht aus der Gruppe der anionischen, nichtionischen, amphoteren oder kationischen Tenside oder geeigneter Mischungen miteinander oder untereinander enthalten ist.

8. Dekontaminationslösung gemäß mindestens einem der Ansprüche 1 bis 7, **dadurch gekennzeichnet, dass** das Metallion gemäß b) in Mengen von 5 mM bis 10 mM enthalten ist.

9. Dekontaminationslösung gemäß mindestens einem der Ansprüche 1 bis 8, **dadurch gekennzeichnet, dass** die oberflächenaktive Substanz gemäß c) in Mengen von 0.2 % bis 0.5 Gew.-% der Gesamtlösung enthalten ist.

10. Nicht-therapeutische Verwendung der Lösungen gemäß mindestens einem der Ansprüche 1 bis 9 zur Denaturierung, Modifikation, Degradation, Solubilisierung und Entfernung von Proteinen und Nukleinsäuremolekülen von Oberflächen.

11. Verfahren zum Einstellen des pH-Wertes einer Dekontaminationslösung gemäß mindestens einem der Ansprüche 1 bis 9, **dadurch gekennzeichnet, dass** die Mischung mittels eines Puffersystems umfassend Carbonate und Derivate der Bernsteinsäure auf einen pH-Wert im Bereich von 2 bis 8.5 eingestellt wird.

## Claims

1. Decontamination solution at least including a synergistic mixture of
a) at least one vitamin in concentrations from 10 mM to 100 mM,
b) at least one metal ion in concentrations from 1 mM to 100 mM, and
c) at least on surface-active substance in concentrations from 0.1 % to 10 % (weight) in relation to the total solution.

2. Decontamination solution according to claim 1, **characterized in that** the mixture has a pH value ranging between pH 2 and 8.5.

3. Decontamination solution according to claim 1 or 2, **characterized in that** the mixture additionally includes a buffer system including carbonates and derivatives of succinic acid.

4. Decontamination solution according to at least one of claims 1 to 3, **characterized in that** said vitamins, their related salts or acidic derivates are at least one compound selected from the group consisting of water-soluble vitamins having the characteristics of an antioxidant.

5. Decontamination solution according to at least one of claims 1 to 4, **characterized in that** said metal ions are taken from the 4^{th} group and subgroups I, II, or VIII of the periodic table of the elements.

6. Decontamination solution according to claim 5, **characterized in that** said metals are salts from their respective acids or bases.

7. Decontamination solution according to at least one of claims 1 to 6, **characterized in that** said surface-active substance is at least one compound selected from the group consisting of anionic, non-ionic, amphoteric or cationic tensides and suitable mixtures thereof.

8. Decontamination solution according to at least one of claims 1 to 7, **characterized in that** said metal ion according to b) is included in concentrations from 5 mM to 10 mM.

9. Decontamination solution according to at least one of claims 1 to 8, **characterized in that** said surface-active substance according to c) is included in concentrations from 0.2 % to 0.5 % (weight) in relation to the total solution.

10. Non-therapeutic use of the decontamination solutions according to at least one of claims 1 to 9 for denaturation, modification, degradation, solubilisation and removal of proteins and nucleic acid molecules from surfaces.

11. Method for adjusting the pH value of a decontamination solution according to at least one of claims 1 to 9, **characterized in that** the mixture is adjusted by a buffer system comprising carbonates and derivatives of succinic acid to a pH value ranging between 2 to 8.5.

## Revendications

1. Solution de décontamination, contenant au moins un mélange synergique composé
a) d'au moins une vitamine dans des quantités comprises entre 10 mM et 100 mM,
b) d'au moins un ion métallique dans des quantités comprises entre 1 mM et 100 mM, et
c) d'au moins un composé tensioactif dans des quantités comprises entre 0,1 % et 10 % en poids, par rapport à la solution dans son ensemble.

2. Solution de décontamination selon la revendication 1, **caractérisée en ce que** le mélange présente un pH compris entre 2 et 8,5.

3. Solution de décontamination selon les revendications 1 ou 2, **caractérisée en ce que** le mélange contient, en outre, un système de tampon avec des carbonates et des dérivés de l'acide succinique.

4. Solution de décontamination selon au moins une des revendications 1 à 3, **caractérisée en ce qu'**elle contient, à titre de vitamines, de leurs sels ou leurs dérivés acides, au moins un composé choisi dans le groupe des vitamines hydrosolubles ayant des propriétés d'antioxydants.

5. Solution de décontamination selon au moins une des revendications 1 à 4, **caractérisée en ce que** les ions métalliques sont issus des métaux de la période 4 et des sous-groupes I, II ou VIII du tableau périodique des éléments.

6. Solution de décontamination selon la revendication 5, **caractérisée en ce que** les métaux se présentent sous forme de leurs sels avec des acides ou des bases.

7. Solution de décontamination selon au moins une des revendications 1 à 6, **caractérisée en ce qu'**elle contient, à titre de substance tensioactive, au moins un composé choisi dans le groupe des tensioactifs anioniques, non-ioniques, amphotères ou cationiques ou de tout mélanges approprié qu'ils forment entre eux.

8. Solution de décontamination selon au moins une des revendications 1 à 7, **caractérisée en ce qu'**elle contient ledit ion métallique selon b) dans des quantités comprises entre 5 mM et 10 mM.

9. Solution de décontamination selon au moins une des revendications 1 à 8, **caractérisée en ce qu'**elle contient ladite substance tensioactive selon c) dans des quantités comprises entre 0,2 % et 0,5 % en poids, par rapport à la solution dans son ensemble.

10. Utilisation non-thérapeutique des solutions selon au moins une des revendications 1 à 9 pour la dénaturation, modification, dégradation, solubilisation et élimination de protéines et de molécules d'acide nucléique qui sont présentes sur des surfaces.

11. Procédé d'ajustement du pH d'une solution de décontamination selon un au moins une des revendications 1 à 9, **caractérisé en ce que** ledit mélange est ajusté à un pH compris entre 2 et 8,5 au moyen d'un système de tampon comprenant des carbonates et des dérivés de l'acide succinique.
